(19) Europäisches Patentamt / European Patent Office / Office européen des brevets

(11) EP 4 038 631 B1

(12) **EUROPEAN PATENT SPECIFICATION**

(45) Date of publication and mention
of the grant of the patent:
**02.07.2025 Bulletin 2025/27**

(21) Application number: **20797251.4**

(22) Date of filing: **30.09.2020**

(51) International Patent Classification (IPC):
*G16H 50/20* (2018.01)      *G16H 20/40* (2018.01)

(52) Cooperative Patent Classification (CPC):
**G16H 50/20; G16H 20/40**

(86) International application number:
**PCT/US2020/053384**

(87) International publication number:
**WO 2021/067343 (08.04.2021 Gazette 2021/14)**

(54) **CASCADE OF MACHINE LEARNING MODELS TO SUGGEST IMPLANT COMPONENTS FOR USE IN ORTHOPEDIC JOINT REPAIR SURGERIES**

KASKADE MASCHINELLER LERNMODELLE ZUM VORSCHLAGEN VON IMPLANTATKOMPONENTEN ZUR VERWENDUNG IN ORTHOPÄDISCHEN GELENKREPARATUR-OPERATIONEN

CASCADE DE MODÈLES D'APPRENTISSAGE MACHINE POUR SUGGÉRER DES COMPOSANTS D'IMPLANTS À UTILISER DANS DES CHIRURGIES ORTHOPÉDIQUES DE RÉPARATION DES ARTICULATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2019 US 201962910110 P**

(43) Date of publication of application:
**10.08.2022 Bulletin 2022/32**

(73) Proprietor: **Howmedica Osteonics Corp.**
**Mahwah, NJ 07430 (US)**

(72) Inventor: **CHAOUI, Jean**
**29280 Plouzané (FR)**

(74) Representative: **Zimmermann & Partner**
**Patentanwälte mbB**
**Postfach 330 920**
**80069 München (DE)**

(56) References cited:
**WO-A1-2015/068035      US-A1- 2019 146 458**

• **ANONYMOUS: "Cascading classifiers - Wikipedia", WIKIPEDIA, 17 September 2019 (2019-09-17), XP055756880, Retrieved from the Internet <URL:https://en.wikipedia.org/w/index. php?title=Cascading_classifiers& oldid=916245602> [retrieved on 20201204]**

**Description**

This application claims the benefit of U.S. Provisional Patent Application 62/910,110, filed October 3, 2019,

**BACKGROUND**

Orthopedic joint repair surgeries involve repair and/or replacement of a damaged or diseased joint. Many times, an orthopedic joint repair procedure, such as joint arthroplasty as an example, involves replacing the damaged joint with an implant component that is attached to a patient's bone. There are numerous challenges associated with planning orthopedic joint repair surgeries. For example, selection of implant components that are appropriately sized and shaped to ensure an optimal surgical outcome can be challenging. US 2019/146458 A1 relates to systems and methods for assisting a surgeon and producing patient-specific medical devices.

**SUMMARY**

This disclosure describes a variety of techniques for providing pre-operative and intra-operative planning and guidance for orthopedic joint repair surgeries. The techniques may be used independently or in various combinations to support particular phases or settings for orthopedic joint repair surgeries. In various examples, the disclosure describes techniques for using machine learning models, such as artificial neural networks, to recommend surgical items for use orthopedic joint repair surgeries. As described herein, a computing system may implement a cascade of machine learning models to recommend surgical items for use in orthopedic joint repair surgeries.

In one example, this disclosure describes a computer-implemented method for determining a suggested implant component to be implanted into a patient during an orthopedic surgery, the method comprising: applying, by a computing system, a first machine learning model to determine a suggested pathology, wherein an input vector of the first machine learning model includes a set of input data, the set of input data including anatomic parameters of the patient; applying, by the computing system, a second machine learning model to determine a suggested surgery, wherein an input vector of the second machine learning model includes an element that indicates the suggested pathology; applying, by the computing system, a third machine learning model to determine the suggested implant component to implant into the patient during the orthopedic surgery, wherein an input vector of the third machine learning model includes an element that indicates the suggested pathology and an element that indicates the suggested surgery; and outputting, by the computing system, an indication of the suggested implant component.

In another example, this disclosure describes a computing system configured to determine a suggested implant component to be implanted into a patient during an orthopedic surgery, the computing system comprising: a data storage system configured to store parameters of a first machine learning model, a second machine learning model, and a third machine learning model; and one or more processing circuits configured to: apply the first machine learning model to determine a suggested pathology, wherein an input vector of the first machine learning model includes a set of input data, the set of input data including anatomic parameters of the patient; apply the second machine learning model to determine a suggested surgery, wherein an input vector of the second machine learning model includes an element that indicates the suggested pathology; apply the third machine learning model to determine the suggested implant component to implanted into the patient during the orthopedic surgery, wherein an input vector of the third machine learning model includes an element that indicates the suggested pathology and an element that indicates the suggested surgery; and output an indication of the suggested implant component.

In another example, this disclosure describes a computing system for determining a suggested implant component to be implanted into a patient during an orthopedic surgery, the computing system comprising: means for applying a first machine learning model to determine a suggested pathology, wherein an input vector of the first machine learning model includes a set of input data, the set of input data including anatomic parameters of the patient; means for applying a second machine learning model to determine a suggested surgery, wherein an input vector of the second machine learning model includes an element that indicates the suggested pathology; means for applying a third machine learning model to determine the suggested implant component to implant into the patient during the orthopedic surgery, wherein an input vector of the third machine learning model includes an element that indicates the suggested pathology and an element that indicates the suggested surgery; and means for outputting an indication of the suggested implant component.

In another example, this disclosure describes a computer-readable data storage medium comprising instructions configured to cause one or more processors to apply a first machine learning model to determine a suggested pathology, wherein an input vector of the first machine learning model includes a set of input data, the set of input data including anatomic parameters of the patient; apply a second machine learning model to determine a suggested surgery, wherein an input vector of the second machine learning model includes an element that indicates the suggested pathology; apply a third machine learning model to determine the suggested implant component to implant into the patient during the orthopedic surgery, wherein an input vector of the third machine learning model includes an element that indicates the suggested pathology and an element that indicates the suggested surgery; and output an indication of the suggested implant component.

The details of various examples of the disclosure are set forth in the accompanying drawings and the description below. Various features, objects, and advantages will be apparent from the description, drawings, and claims.

## BRIEF DESCRIPTION OF THE DRAWINGS

FIG. 1 is a block diagram illustrating an example system that implements an AI system for suggesting a pathology of a patient, suggesting a surgery for the patient, and/or suggesting implant component types, in accordance with a technique of this disclosure.

FIG. 2 is a conceptual block diagram illustrating an example system for suggesting a pathology, surgery, and/or implant components for a patient, in accordance with one or more aspects of this disclosure.

FIG. 3 illustrates an example neural network that may be implemented by a computing system with the system of FIG. 2.

FIG. 4 is a flowchart illustrating an example operation for determining a suggested pathology, suggested surgery, and/or suggested implant component type, in accordance with one or more aspects of this disclosure.

## DETAILED DESCRIPTION

Certain examples of this disclosure are described with reference to the accompanying drawings, wherein like reference numerals denote like elements. It should be understood, however, that the accompanying drawings illustrate only the various implementations described herein and are not meant to limit the scope of various technologies described herein. The drawings show and describe various examples of this disclosure. In the following description, numerous details are set forth to provide an understanding of the techniques of this disclosure. However, it will be understood by those skilled in the art that the techniques of this disclosure may be practiced without these details and that numerous variations or modifications from the described examples may be possible.

Orthopedic surgery can involve implanting one or more implant components into a patient to repair or replace a damaged or diseased joint of the patient. Today, surgical planning tools are available that use image data of the diseased or damaged joint to generate an accurate three-dimensional bone model that can be viewed and manipulated preoperatively by the surgeon. These surgical planning tools can enhance surgical outcomes by allowing the surgeon to simulate the orthopedic surgery, select or design implant components that more closely match the contours of the patient's actual bone, and select or design surgical instruments and guide tools that are adapted specifically for repairing the bone of a particular patient. Use of these planning tools may result in generation of a preoperative surgical plan, complete with implant components and surgical instruments that are selected or manufactured for the individual patient.

When planning an orthopedic surgery, the surgeon may have a plethora of implant components from which to select. For instance, in an example where the surgeon is planning a shoulder repair surgery, the surgeon may select from among various stemmed humeral implants, various stemless humeral implants, various wedged glenoid implants, various non-wedged glenoid implants, various glenoid platform implants, various humeral platform implants, and so on. Selecting the right implant components may be the difference between success and failure of the orthopedic surgery. For instance, selecting a glenoid implant component or humeral implant component that is too large or too small, having an incorrect shape, or having one or more other inappropriate aspects, may impact whether the patient recovers a full range of motion. In some instances, incorrect selection of implant components may lead to loosening of the implant components, which may lead to performance of an orthopedic revision surgery. Less experienced surgeons may struggle with how to select the correct implant components among the large number of available implant components available.

This disclosure describes techniques for using artificial intelligence (AI) techniques to suggest implant components for use in orthopedic joint repair surgeries. The AI techniques of this disclosure may be implemented in a surgical planning platform, such as the BLUEPRINT™ software system available from Wright Medical Technology, Inc. There have been several challenges associated with application of AI systems to planning orthopedic surgeries, particularly with respect to shoulder pathology. For example, challenges relate to structuring and training AI systems so that the AI systems are able to provide accurate and valuable suggestions for implant components.

In accordance with one or more techniques of this disclosure, a computing system implements a cascade of machine learning models, such as artificial neural networks (ANNs), to suggest implant components for use in orthopedic joint repair surgeries. For example, a computing system may apply a first machine learning model to determine a suggested pathology. In this example, an input vector of a machine learning model is or includes a set of input data that the machine learning model may use to generate a prediction or other type of output, such as a suggested pathology. The set of input data includes anatomic parameters of the patient. Furthermore, in this example, the computing system may apply a second machine learning model to determine a suggested surgery. In this example, an input vector of the second machine learning model includes an element that indicates the suggested pathology. Additionally, in this example, the computing system may apply a third machine learning model to determine the suggested implant component to implant into the patient during the orthopedic surgery. In this example, an input vector of the third machine learning model includes an element that indicates the suggested pathology and an element that indicates the suggested surgery. The computing system may output an indication of the suggested implant component. Using this cascade of machine learning models may produce more accurate suggestions for implant components.

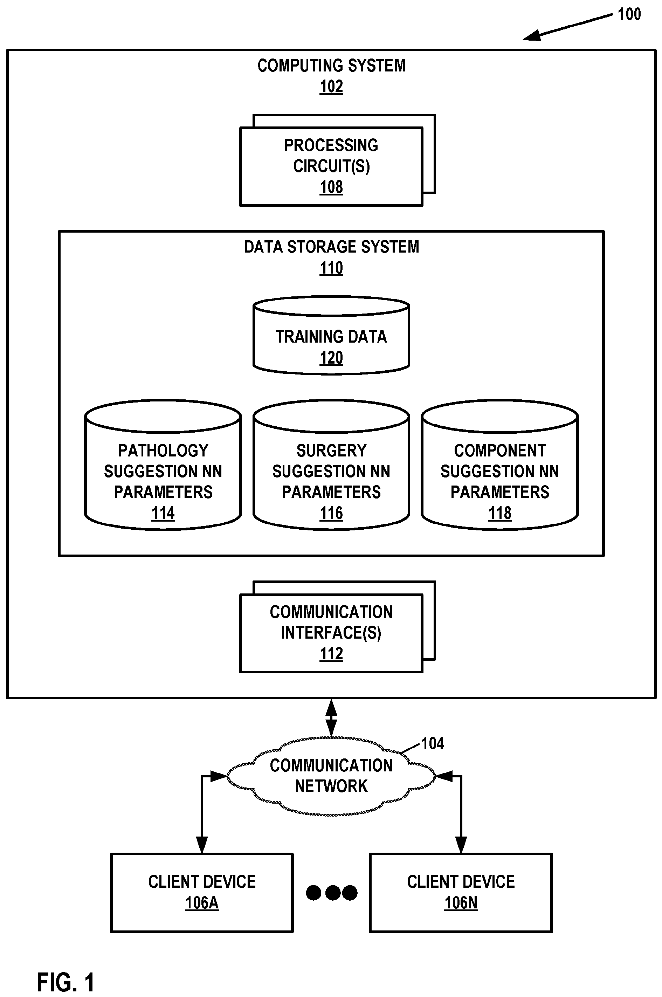

FIG. 1 is a block diagram illustrating an example system 100 that implements an AI system for suggesting a pathology of a patient, suggesting a surgery for the patient, and/or suggesting one or more implant component types, in accordance with a technique of this disclosure. As described in this disclosure, computing system 102 may use a cascade of machine learning models, such as neural networks or other types of machine learning models, to suggest implant components for use in orthopedic joint repair surgeries.

As shown in the example of FIG. 1, system 100 includes a computing system 102, a communication network 104, a set of one or more client devices (collectively, "client devices 106"). In other examples, system 100 may include more, fewer, or different devices and systems. In the example of FIG. 1, computing system 102 and client devices 106 communicate via communication network 104, such as the Internet.

Computing system 102 may include one or more computing devices. Computing system 102 and client devices 106 may include various types of computing devices, such as server computers, personal computers, smartphones, laptop computers, mixed reality (MR) or augmented reality (AR) visualization devices, virtual reality (VR) visualization devices, and other types of computing devices. In the example of FIG. 1, computing system 102 includes one or more processing circuits 108, a data storage system 110, and a set of one or more communication interfaces 112. Data storage system 110 is configured to store data. Communication interface(s) 112 may enable computing system 102 to communicate (e.g., wirelessly or using wires) to other computing systems and devices, such as client devices 106. For ease of explanation, this disclosure may describe actions performed by processing circuits 108, data storage system 110, and communication interface(s) 112 as being performed by computing system 102 as a whole.

Users may use client devices 106 to access information generated by computing system 102. Because computing system 102 may be remote from client devices 106, users of client devices 106 may consider computing system 102 to be a cloud-based computing system. In other examples, some or all the functionality of computing system 102 may be performed by one or more of client devices 106.

Planning an orthopedic surgery, such as a shoulder arthroplasty, may involve determining a pathology of a patient, selecting an orthopedic surgery to address the pathology, and selecting a set of implant components that will be used during the selected orthopedic surgery. In examples involving a shoulder of a patient, example pathologies may include primary glenoid humeral osteoarthritis (PGHOA), rotator cuff tear arthropathy (RCTA), instability, massive rotator cuff tear (MRCT), rheumatoid arthritis, post-traumatic arthritis, osteoarthritis, and so on.

Example orthopedic surgeries may include a stemless standard total shoulder arthroplasty, a stemmed standard total shoulder arthroplasty, a stemless reverse shoulder arthroplasty, a stemmed reverse shoulder arthroplasty, an augmented glenoid standard total shoulder arthroplasty, an augmented glenoid reverse shoulder arthroplasty, a shoulder hemiarthroplasty, and other types of orthopedic surgeries to repair a patient's shoulder joint. Example types of implant components may include differently sized glenoid protheses, humeral protheses with differently sized stems, prostheses with different shapes or angles, and so on.

The set of implant components that will be used during an orthopedic surgery may vary from patient to patient. Selecting an appropriate set of implant components for a specific patient may be challenging for a surgeon, especially if the surgeon does not perform the orthopedic surgery on a regular basis. Accordingly, it may be desirable to implement an automated process for recommending implant components to a surgeon.

This disclosure describes techniques and systems that implement an automatic process for suggesting implant components to a surgeon. As described herein, computing system 102 may implement a cascade of multiple artificial neural networks (ANN) (or other types of machine learning models) to recommend implant components to a surgeon. In some examples, the ANNs may be separately trained. An ANN may include an input layer, an output layer, and one or more hidden layers between the input layer and the output layer. ANNs may also include one or more other types of layers, such as pooling layers. Each layer on an ANN may include a set of artificial neurons, which are frequently referred to simply as "neurons." The input layer includes a plurality of input layer neurons. Each input layer neuron in the plurality of input layer neurons corresponds to a different input element in an input vector. Outputs of the neurons in the input layer are provided as inputs to a next layer in the ANN. Each neuron of a layer after the input layer may apply a propagation function to the output of one or more neurons of the previous layer to generate an input value to the neuron. The propagation function computes a weighted sum of outputs of predecessor neurons. The following equations are example propagation functions:

$$p_j(t) = \sum_i o_i(t)w_{ij} \qquad \text{(Eq. 1)}$$

$$p_j(t) = \sum_i o_i(t)w_{ij} + w_{0j} \qquad \text{(Eq. 2)}$$

In Eq. 1 and Eq. 2, $p_j(t)$ is an input to a neuron at time $t$, $i$ an index of the predecessor neurons to the neuron, $o_i(t)$ is output of predecessor neuron $i$ at time $t$, $w_{ij}$ is a weight for the predecessor neuron $i$, and $w_{0j}$ is a bias term.

The neuron may apply an activation function to the input to compute an activation value. Example activation functions may include sigmoid activation functions, rectified linear unit (ReLU) activation functions, a TanH activation function, and so on. The neuron may then apply an output function to the activation value to generate an output value for the neuron. In some examples, the output function is an identity function. The output layer includes a plurality of output layer neurons. An output vector of an ANN includes the output values of the output layer of the ANN. Each output layer neuron in the plurality of output layer neurons corresponds to a different output element in a plurality of output elements. Applying a neural network to input data comprises providing the input data to input layer neurons of the neural network and, for each neuron of the neural network in a layer after the input layer, calculating an output value of the neuron based on output values of predecessor neurons.

As indicated above, computing system 102 may implement a cascade of multiple, separately trained ANNs to recommend implant components to a surgeon. The cascade of ANNs may include a first neural network, a second neural network, and at least one third neural network. The first neural network (i.e., a pathology suggestion neural network) suggests a pathology, the second neural network (i.e., a surgery suggestion neural network) suggests a surgery, and at least one third neural network (i.e., at least one component suggestion neural network) suggests one or more implant components for use in an orthopedic surgery on a particular patient. The input vectors of each of the second and third neural networks may include a complete input vector of the first neural network.

Implementing the automatic process for recommending implant components using a cascade of multiple, separately trained neural networks has been found to improve the accuracy and the training speed of an automatic process for suggesting implant components to a surgeon, relative to a single neural network that takes the same input vector as the first neural network and suggests implant components. In other words, for the same input vector, the cascade of neural networks may be more efficient to train and may produce better results than a single neural network. Moreover, providing the suggested pathology to the second and third neural networks and providing the suggested surgery to the third neural networks may improve the accuracy of suggestions produced by those neural networks.

In the example of FIG. 1, data storage system 110 may include pathology suggestion neural network (NN) parameters 114, surgery suggestion neural network parameters 116, and component suggestion neural network parameters 118. Parameters 114 include weights for neurons of the pathology suggestion neural network. Parameters 116 include weights for neurons of the surgery suggestion neural network. Parameters 118 include weights for neurons of the component suggestion neural network. Parameters 114, 116, 118 may also include other values, such as bias values, that control the output of the pathology suggestion neural network, the surgery suggestion neural network, and the component suggestion neural network.

As noted above, the pathology suggestion neural network, the surgery suggestion neural network, and the component suggestion neural network may be separately trained. In other words, each of these neural networks goes through a training process that is independent of the training processes used to train other ones of these neural networks. For instance, computing system 102 may train the pathology suggestion neural network, the surgery suggestion neural network, and the component suggestion neural network separately from each other. When computing system 102 trains the pathology suggestion neural network, computing system 102 may perform a training process that updates the values of weights of the pathology suggestion neural network based on error values indicative of differences between likelihood values output by the pathology suggestion neural network for various pathologies and target output values for the pathologies. Similarly, computing system 102 may perform a training process that updates the values of weights of the surgery suggestion neural network based on error values indicative of differences between likelihood values output by the surgery suggestion neural network for various surgeries and target output values for the surgeries. Likewise, computing system 102 may perform a training process that updates the values of weights of a component suggestion neural network based on error values indicative of differences between likelihood values output by the component suggestion neural network for various implant components and target output values for the implant components.

In the example of FIG. 1, data storage system 110 may store training data 120. As described in greater detail elsewhere in this disclosure, computing system 102 may use training data 120 to train the pathology suggestion neural network, the surgery suggestion neural network, and the component suggestion neural network. Training data 120 include training datasets regarding past orthopedic surgery cases. In some examples, each respective training dataset corresponds to a different training data patient in a plurality of training data patients and comprises a respective training input vector and a respective target output vector.

For each respective training dataset, the training input vector of the respective training dataset comprises a value for each element of a plurality of input elements. For each respective training dataset, the target output vector of the respective training dataset comprises a value for each element of the plurality of output elements. In this example, computing system 102 may use the plurality of training datasets to train the pathology suggestion neural network, surgery suggestion neural networks, and component suggestion neural networks.

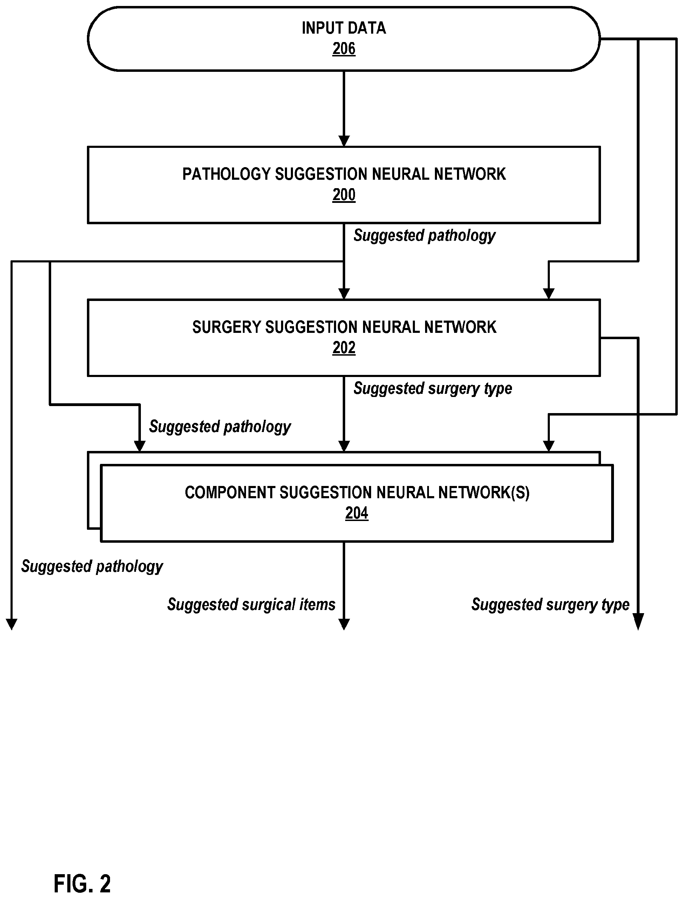

FIG. 2 is a conceptual block diagram illustrating an example system for suggesting a pathology, surgery, and implant components for a patient, in accordance with one or more aspects of this disclosure. In the example of FIG. 2, computing system 102 implements a pathology suggestion neural network 200, a surgery suggestion neural network 202, and one or more component suggestion neural networks 204.

Pathology suggestion neural network 200 is configured to suggest a pathology based on a set of input data 206 for a patient. For instance, in one example, the suggested pathologies may include one or more of primary glenoid humeral osteoarthritis (PGHOA), rotator cuff tear arthropathy (RCTA) instability, massive rotator cuff tear (MRCT), rheumatoid arthritis, post-traumatic arthritis, and osteoarthritis.

Pathology suggestion neural network 200 has an input layer, an output layer, and one or more hidden layers between the input layer and the output layer. For instance, in one example, pathology suggestion neural network 200 has two hidden layers between the input layer and the output layer. Each layer of pathology suggestion neural network 200 includes a plurality of artificial neurons. For instance, a first hidden layer may include 25 artificial neurons and a second hidden layer may include 15 artificial neurons. The artificial neurons in the input layer of pathology suggestion neural network 200 may include a respective artificial neuron for each element of an input vector of pathology suggestion neural network 200. The input vector of pathology suggestion neural network 200 may include each element of input data 206.

The output layer of pathology suggestion neural network 200 may include a plurality of artificial neurons. The artificial neurons in the output layer of pathology suggestion neural network 200 include a respective artificial neuron that outputs a value of a respective element of an output vector of pathology suggestion neural network 200. Each element of the output vector of pathology suggestion neural network 200 may correspond to a different potential pathology. Thus, different neurons in the output layer of pathology suggestion neural network 200 correspond to different pathologies in a plurality of pathologies. The plurality of pathologies may include primary glenoid humeral osteoarthritis, rotator cuff tear arthropathy instability, massive rotator cuff tear, rheumatoid arthritis, post-traumatic arthritis, osteoarthritis, and so on. In some examples, for each element of the output vector of pathology suggestion neural network 200, the value of the element indicates a likelihood that the values in the input vector of pathology suggestion neural network 200 correspond to the potential pathology. For instance, the value of the element may indicate a probability or a value corresponding to a level of confidence that the values in the input vector of pathology suggestion neural network 200 correspond to the potential pathology. Computing system 102 may determine that the suggested pathology is the potential pathology with the greatest likelihood. In some examples, computing system 102 may also output indications of the likelihoods of the potential pathologies, including the suggested pathology and, in some examples, the other potential pathologies.

Surgery suggestion neural network 202 is configured to suggest a surgery based on the suggested pathology and input data 206 for the patient. For instance, in one example, the suggested surgery may be a type of shoulder surgery. In this example, the suggested surgery may be a stemless standard total shoulder arthroplasty, a stemmed standard total shoulder arthroplasty, a stemless reverse shoulder arthroplasty, a stemmed reverse shoulder arthroplasty, an augmented glenoid standard total shoulder arthroplasty, or an augmented glenoid reverse shoulder arthroplasty. In other examples, the suggested surgery may be a standard shoulder arthroplasty or a reverse shoulder arthroplasty.

Surgery suggestion neural network 202 has an input layer, an output layer, and one or more hidden layers between the input layer and the output layer. For instance, in one example, surgery suggestion neural network 202 has two hidden layers between the input layer and the output layer. The input layer of surgery suggestion neural network 202 may include a plurality of artificial neurons. The artificial neurons in the input layer of surgery suggestion neural network 202 include a respective artificial neuron for each element of an input vector of surgery suggestion neural network 202. The input vector of surgery suggestion neural network 202 may include each element of input data 206 and an element indicating the suggested pathology. Thus, the determination of the suggested pathology may be based in part on the suggested pathology of the patient.

The output layer of surgery suggestion neural network 202 may include a plurality of artificial neurons. The artificial neurons in the output layer of surgery suggestion neural network 202 include a respective artificial neuron that outputs a value of a respective element of an output vector of surgery suggestion neural network 202. Each element of the output vector of surgery suggestion neural network 202 may correspond to a different potential surgery. For instance, a first element of the output vector may correspond to a standard shoulder arthroplasty and a second element of the output vector may correspond to a reverse shoulder arthroplasty. Thus, different neurons in an output layer of surgery suggestion neural network 202 may correspond to different orthopedic surgeries in a plurality of orthopedic surgeries. The plurality of orthopedic surgeries includes two or more of: anatomical total shoulder arthroplasty, reverse total shoulder arthroplasty, or shoulder hemiarthroplasty.

In some examples, for each element of the output vector of surgery suggestion neural network 202, the value of the element indicates a likelihood that the values in the input vector of surgery suggestion neural network 202 correspond to the potential surgery. For instance, the value of the element may indicate a probability or a value corresponding to a level of confidence that the values in the input vector of surgery suggestion neural network 202 correspond to the potential surgery. Computing system 102 may determine that the suggested surgery is the potential surgery with the greatest likelihood. In some examples, computing system 102 may also output indications of the likelihoods of the potential surgeries, including the suggested surgery and, in some examples, the other potential surgeries.

As noted above, computing system 102 may implement one or more component suggestion neural networks 204. Each of component suggestion neural networks 204 may correspond to a different implant component type. In this disclosure, an implant component type is a set of implant components that fulfill the same functional role during or after the orthopedic surgery. By using different component suggestion neural networks for different implant component types, each of the component suggestion neural networks may be trained more directly to select an appropriate implant component within an implant component type, rather than training a single neural network to concurrently identify the most appropriate implant components within a plurality of implant component types. Each of component suggestion neural networks 204 is configured to suggest one or more implant components within the corresponding implant component type. Each of component suggestion neural networks 204 may receive, as input, input data 206 for the patient, data indicating the suggested pathology, and data indicating the suggested surgery. In some examples, component suggestion neural networks 204 may receive, as input, input data in addition to input data 206. For example, component suggestion neural networks 204 may receive input data indicating additional anatomic parameters, such as one or more of humeral head medial offset, humeral head lateral offset, internal diameter of diaphysis, external diameter of diaphysis, and so on.

Thus, in some examples, computing system 102 may apply a first component suggestion neural network to determine a first suggested implant component to be implanted into a patient during an orthopedic surgery and may also apply a second component suggestion neural network to determine a second suggested implant component to be implanted into the patient during the orthopedic surgery. In such examples, the second component suggestion neural network is separate from the first component suggestion neural network. Furthermore, in such examples, the first suggested implant component belongs to a first implant component type and the second suggested implant component belongs to a second implant component type. In such examples, the first and second suggested implant components may be designed for attachment to different bones of the patient. For instance, in an example where the orthopedic surgery is a shoulder repair surgery, the first suggested implant component may be a glenoid implant and the second suggested implant component may be a humeral implant component.

As mentioned above, an implant component type is a set of implant components that fulfill the same functional role during or after the orthopedic surgery. For instance, in an example involving shoulder repair surgery, a first one of component suggestion neural networks 204 may correspond to a set of humeral implant components, a second one of component suggestion neural networks 204 may correspond to a set of glenoid implant components, a third one of component suggestion neural networks 204 may correspond to a set of bone grafts to be grafted between the patient's glenoid and the glenoid implant, and so on. In this example, the set of humeral implant components may include various implant components that are to be attached to the patient's humerus. For instance, the set of humeral implant components may include a set of stemmed humeral implants and a set of stemless humeral implants. The set of stemmed humeral implants may include stemmed humeral implants having different sizes and shapes. Likewise, the set of stemless humeral implants may include stemless humeral implants having different sizes and shapes.

The set of glenoid implant components may include various implant components that are to be attached to the patient's glenoid. For instance, the set of glenoid implant components may include a set of wedged-platform glenoid implants, a set of non-wedged-platform glenoid implants, a set of pegged glenoid implants, and a set of keeled glenoid implants. The set of wedged-platform glenoid implants may include wedged-platform glenoid implants having various sizes and shapes. The set of non-wedged-platform glenoid implants may include non-wedged-platform glenoid implants having various sizes and shapes. Platform glenoid implants are glenoid implants that may serve as platforms for either cup-shaped glenoid implant components in anatomical total shoulder arthroplasties or glenospheres for reverse total shoulder arthroplasties. Each of these sets within the set of glenoid implant components may include glenoid implants having various sizes and shapes.

Different sizes and shapes of implant components (e.g., humerus implant components and glenoid implant components) may be used for different patients. For instance, in a standard total shoulder arthroplasty, a surgeon may attach a glenoid prothesis to a patient's scapula. One side of the glenoid prothesis is in contact with the patient's scapula and an opposite side of the glenoid prothesis defines a cup in which a head of a humeral prothesis may slide. Because different patients may have differently shaped scapulae and/or different types of bone loss, the scapula-facing sides of glenoid protheses may need to have different shapes. For instance, the scapula-facing sides of glenoid protheses may need to have no wedge or wedges of different thicknesses in order to accommodate for different amounts of asymmetric bone loss. Accordingly, the recommended implant component may be a glenoid prothesis having a correct shape for a particular patient. In some examples, the recommended implant component may be further customized.

Each of component suggestion neural networks 204 has an input layer, an output layer, and one or more hidden layers between the input layer and the output layer. For instance, in one example, each of component suggestion neural networks 204 has two hidden layers between the input layer and the output layer. The input layer of each of component suggestion neural networks 204 may include a plurality of artificial neurons. For each of component suggestion neural networks 204, the artificial neurons in the input layer of the component suggestion neural network includes a respective artificial neuron for each element of an input vector of the component suggestion neural network.

For each of component suggestion neural networks 204, the input vector of the component suggestion neural network may include each element of input data 206, an element indicating the suggested pathology, and an element indicating the suggested surgery. The output layer of each of component suggestion neural networks 204 may include a plurality of artificial neurons. The artificial neurons in the output layer of a component suggestion neural network includes a respective artificial neuron that outputs a value of a respective element of an output vector of the component suggestion neural network. Each element of the output vector of a component suggestion neural network may correspond to a different implant component. For instance, a first element of the output vector may correspond to a first type of stemmed humeral implant, a second element of the output vector may correspond to a second type of stemmed humeral implant, a third element of the output vector may correspond to a first type of stemless humeral implant, a fourth element of the output vector may correspond to a second type of stemless humeral implant, and so on. Thus, different neurons in the output layer of a component suggestion neural network may correspond to different implant components. In some examples, different output neurons of the component suggestion neural network may correspond to different combinations of sizes and shapes for one or more different types of implant components. In some examples, for each element of the output vector of a component suggestion neural network, the value of the element indicates a likelihood that the corresponding implant component should be used, given values in the input vector of the component suggestion neural network.

Because the input vectors of component suggestion neural networks 204 include elements indicating the suggested surgery, component suggestion neural networks 204 may assign comparatively low likelihood values (e.g., 0) to implant components that are incompatible with the suggested surgery. For example, cup-shaped glenoid implants are incompatible with a reverse total shoulder arthroplasty. Accordingly, in this example, a component suggestion neural network corresponding to glenoid implants may be trained to assign a likelihood value of 0 to the cup-shaped glenoid implants when the suggested surgery is a reverse total shoulder arthroplasty. Furthermore, because the input vectors of component suggestion neural networks 204 include elements indicating the suggested pathology, the component suggestion neural networks 204 may be trained to assign low likelihood values to implant components that are unlikely to be used with the suggested pathology. For example, a non-wedged glenoid implant component may be unlikely to be used when the suggested pathology involves erosion of one rim of the patient's glenoid.

Although the above examples have described component recommendation neural networks corresponding to different implant component types, computing system 102 may implement component recommendation neural networks corresponding to a two or more implant component types or all implant component types.

In some examples, the set of input data 206 provided to pathology suggestion neural network 200, surgery suggestion neural network 202, and component suggestion neural networks 204 may include the same data. Thus, surgery suggestion neural network 202 may have the same input as pathology suggestion neural network 200, plus the suggested pathology. Similarly, component suggestion neural networks 204 may have the same input as surgery suggestion neural network 202, plus the suggested surgery. Reusing input data 206 may simplify the process of training and applying pathology suggestion neural network 200, surgery suggestion neural network 202, and component suggestion neural networks 204. In other examples, the computing system may provide different sets of input data pathology suggestion neural network 200, surgery suggestion neural network 202, and component suggestion neural networks 204.

The set of input data 206 may include various types of data. For instance, in some examples, the set of input data 206 may include data related to anatomic parameters, bony landmark metrics, data related to bony density, and data related to clinical measurements. Anatomic parameters may include indications of the presence of physical anatomic features of the patient or measurements of physical anatomic features of the patient. Examples of anatomic parameters may include parameters corresponding to at least one of: glenoid wear orientation, glenoid bone loss, humeral bone loss, a Hill-Sachs lesion, or a Bankart lesion. In one such example, the clinical measurements may include measurements of retroversion, subluxation, orientation, and so on, or various bones or joints. Bony landmark metrics may be numerical values characterizing distances or angles between landmarks on one or more bones of the patient. Examples of bony landmark metrics include the distance between the patient's humerus and glenoid, a distance between the patient's acromion and humeral head, a glenoid coracoid process angle, an infra-glenoid tubercle angle, and so on. Data related to clinical measurements may include the patient's body mass index (BMI), patient activities, patient profiles, scapula critical shoulder sagittal angle, scapula acromion index, and so on. The anatomic parameters, bony landmark metrics, data related to bony density, data related to clinical measurements, and/or other types of data may be generated or obtained in a variety of ways, including measurements or functions of measurements obtained from medical images, CT models, medical records, and so on.

In some examples, the set of input data 206 may include one or more of the following:

- Surgeon name
- Surgeon identification number
- Case data: any data regarding a case.
- Case operation date: a date on which the surgery is expected to be performed.
- Case age at planification date: the patient's age.
- Case gender: gender of patient.
- Case side: whether the surgery involves the left or right side of the patient.
- Scapula plane: an angle of the scapula in its resting position relative to a midline of the patient's body.
- Scapula critical shoulder angle in degrees - indicates an angle between (i) a line between a top border of the glenoid and a bottom border of the glenoid, and (ii) a line between the bottom border of the glenoid and a most lateral portion of the patient's acromion.
- Scapula critical shoulder sagittal angle in degrees - indicates an angle subtended by a line parallel to a glenoid and a line through an inferior-lateral edge of the glenoid and the inferior-lateral edge of the acromion.
- Acromion humeral space: the space between the acromion and the top of the humerus.
- Scapula acromiohumeral space in millimeters - indicates a space between the acromion and the top of the humeral head.
- Glenoid center: gravity center of the glenoid surface
- Glenoid plane: best fit plane of the glenoid
- Glenoid anterior axis: axis from glenoid center to the anterior tip of the glenoid rim.
- Glenoid lateral axis: transverse axis (Friedman axis)
- Glenoid superior axis: vector product of anterior and lateral axes
- Glenoid sphere center: center of the best fit sphere of the glenoid
- Glenoid sphere radius: radius of the best fit sphere
- Glenoid sphere RMS: root mean squares error of the best fit sphere of the glenoid
- Glenoid area: surface of the glenoid in mm$^2$
- Glenoid inclination - indicates a superior/inferior tilt of the glenoid relative to the scapula.
- Glenoid version - indicates an angular orientation of the axis of the glenoid articular surface relative to the long (transverse) axis of the scapula.
- Glenoid orientation/direction: the 3-dimensional orientation/direction of the glenoid in a 3-dimensional space
- Glenoid reverse shoulder angle: the tilt of the inferior part of the glenoid surface with regard to the vertical.
- Glenoid coracoid process angle in degrees: angle between the coracoid process and the spine
- Glenoid humeral space: the space between the glenoid and the humerus.
- Glenoid inclination: the superior/inferior tile of the glenoid relative to the scapula.
- Glenoid best fit sphere radius: a radius of a best-fit sphere for the patient's glenoid. The best-fit sphere is a conceptual sphere that is sized such that a sector of the sphere would sit flush as possible with the patient's glenoid.
- Glenoid best fit sphere root mean square error: the mean square error of the difference between the patient's glenoid and the sector of the best-fit sphere.
- Reverse shoulder angle: the tilt of the inferior part of the glenoid.
- Humerus direction/orientation: the orientation of the humeral head in a 3-dimensional space. The humerus direction/orientation may be expressed as a polar angle in degrees from 0° to 360°.
- Humeral head best fit sphere radius and root mean square error: a radius of a best-fit sphere for the head of the patient's humerus. The best-fit sphere is a conceptual sphere that is sized such that a sector of the sphere matches the surface of the humeral head as much as possible. The root mean square error indicates the error between the best-fit sphere and the patient's actual humeral head.
- Humeral neck shaft angle: the angle between the humeral anatomic neck normal vector and the intramedullary axis.
- Humerus inclination: indicates an angle of inclination between an articular surface plan and a humeral shaft axis.
- Humerus anatomical neck
- Humerus diaphysis axis
- Humerus subluxation: a measure of the subluxation of the humerus relative to the glenoid.
- Humerus surgical neck
- Humerus Tingart neck
- Humerus Giannotti Cortical Index
- Humerus Tingart Cortical Thickness in millimeters
- Humerus proximal diaphysis density in average Hounsfield units
- Humerus metaphysis spongious density in average Hounsfield units

- Humerus metaphysis cortical density in average Hounsfield units
- Humerus head sphere center
- Humerus head sphere radius
- Humerus head sphere RMS in millimeters
- Humerus to center to glenoid center distance in millimeters
- Humerus version: the angle between the humeral orientation and the epicondylar axis.
- a measurement of an epiphysis of the patient's humerus,
- a measurement of a metaphysis of the patient's humerus,
- a measurement of a diaphysis of the patient's humerus,
- Elbow epicondilar axis - indicates a transepicondylar axis of the elbow.
- Elbow point 1 - indicates a lateral epicondyle characteristic
- Elbow point 2 - indicates a medial epicondyle characteristic
- Glenoid implant type
- Glenoid implant glenosphere type
- Glenoid glenosphere diameter
- Humerus implant head type - indicates a head component type (e.g., constrained, non-constrained, pyrocarbon)

In some examples, input data 206 may include information (e.g., in combination with zero or more other example types of input data described herein) based on a rotator cuff assessment of the patient. For instance, input data 206 may include information, alone or in combination with morphological inputs described above, regarding fatty infiltration of the rotator cuff muscles, atrophy of the rotator cuff muscles, and/or other information about the patient's rotator cuff muscles. In some examples, the information regarding the patient's rotator cuff may be expressed in terms of a class in a shoulder pathology classification system, such as a Warner classification system or a Goutalier classification system.

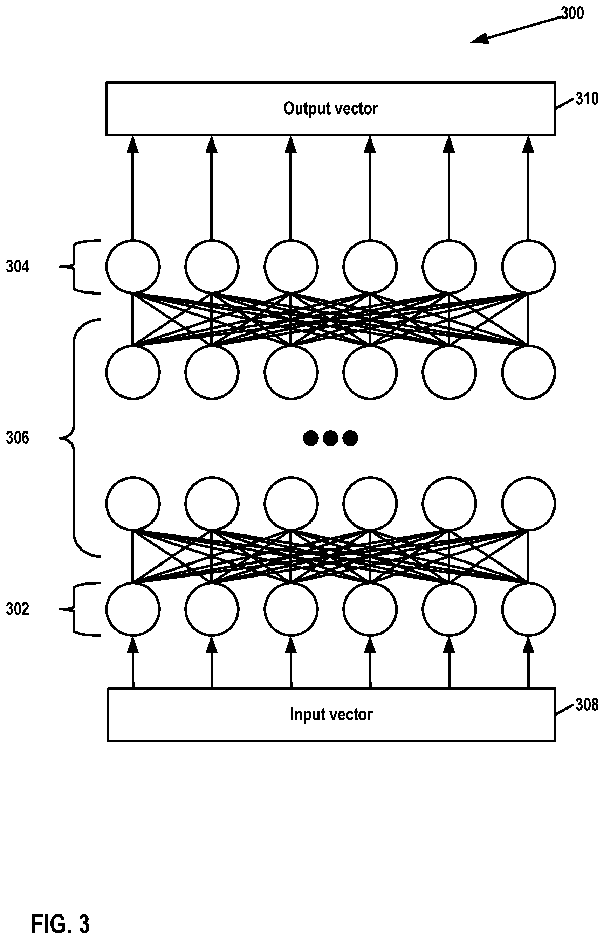

FIG. 3 illustrates an example neural network 300 that may be implemented by computing system 102 with the system of FIG. 2. Pathology suggestion neural network 200, surgery suggestion neural network 202, or component suggestion neural networks 204 may be implemented using a neural network, such as neural network 300. In the example of FIG. 3, neural network 300 includes an input layer 302, an output layer 304, and one or more hidden layers 306 between input layer 302 and output layer 304. In the example of FIG. 3, neurons are represented as circles. Although in the example of FIG. 3, each layer is shown as including six neurons, layers in neural network 300 may include more or fewer neurons. Furthermore, although neural network 300 is shown in FIG. 3 as being a fully connected network, neural network 300 may have a different architecture. For instance, neural network 300 may not be a fully connected network, may have one or more convolutional layers, or may otherwise have a different architecture from that shown in FIG. 3.

In some examples, neural network 300 can be or can include one or more artificial neural networks (also referred to simply as neural networks). A neural network can include a group of connected nodes, which also can be referred to as neurons or perceptrons. A neural network can be organized into one or more layers. Neural networks that include multiple layers can be referred to as "deep" networks. A deep network can include an input layer, an output layer, and one or more hidden layers positioned between the input layer and the output layer. The nodes of the neural network can be connected or non-fully connected.

Neural network 300 can be or can include one or more feed forward neural networks. In feed forward networks, the connections between nodes do not form a cycle. For example, each connection can connect a node from an earlier layer to a node from a later layer.

In some instances, neural network 300 can be or can include one or more recurrent neural networks. In some instances, at least some of the nodes of a recurrent neural network can form a cycle. Recurrent neural networks can be especially useful for processing input data that is sequential in nature. In some instances, a recurrent neural network can pass or retain information from a previous portion of the input data sequence to a subsequent portion of the input data sequence through the use of recurrent or directed cyclical node connections.

In some examples, sequential input data can include time-series data (e.g., sensor data versus time or imagery captured at different times). For example, a recurrent neural network can analyze sensor data versus time to detect or predict a swipe direction, to perform handwriting recognition, etc. Sequential input data may include words in a sentence (e.g., for natural language processing, speech detection or processing, etc.); notes in a musical composition; sequential actions taken by a user (e.g., to detect or predict sequential application usage); sequential object states; etc. Example recurrent neural networks include long short-term (LSTM) recurrent neural networks; gated recurrent units; bi-direction recurrent neural networks; continuous time recurrent neural networks; neural history compressors; echo state networks; Elman networks; Jordan networks; recursive neural networks; Hopfield networks; fully recurrent networks; sequence-to-sequence configurations; etc.

In some implementations, neural network 300 can be or can include one or more convolutional neural networks. In some instances, a convolutional neural network can include one or more convolutional layers that perform convolutions over input data using learned filters. Filters can also be referred to as kernels. Convolutional neural networks can be especially useful for vision problems such as when the input data includes imagery such as still images or video.

Neural network 300 may be or include one or more other forms of artificial neural networks such as, for example, deep Boltzmann machines; deep belief networks; stacked autoencoders; etc. Any of the neural networks described herein can be combined (e.g., stacked) to form more complex networks.

In the example of FIG. 3, an input vector 308 includes a plurality of input elements. Each of the input elements may be a numerical value. Input layer 302 includes a plurality of input layer neurons. Each input layer neuron in the plurality of input layer neurons included in input layer 302 may correspond to a different input element in a plurality of input elements. In other words, input layer 302 may include a different neuron for each input element in input vector 308.

Furthermore, in the example of FIG. 3, an output vector 310 includes a plurality of output elements. Each of the output elements may be a numerical value. Output layer 304 includes a plurality of output layer neurons. Each output layer neuron in the plurality of output layer neurons corresponds to a different output element in the plurality of output elements. In other words, each output layer neuron in output layer 304 corresponds to a different output element in output vector 310.

Input vector 308 may include a wide variety of information. For example, input vector 308 may include any of the types of input data discussed elsewhere in this disclosure. For instance, in examples where pathology suggestion neural network 200 is implemented using neural network 300, input vector 308 may include input data 206. In examples where surgery suggestion neural network 202 is implemented using neural network 300, input vector 308 may include input data 206 and data indicating a suggested pathology. In examples where one or more of component suggestion neural networks 204 are implemented using neural network 300, input vector 308 may include input data 206, data indicating a suggested pathology, and data indicating a suggested surgery.

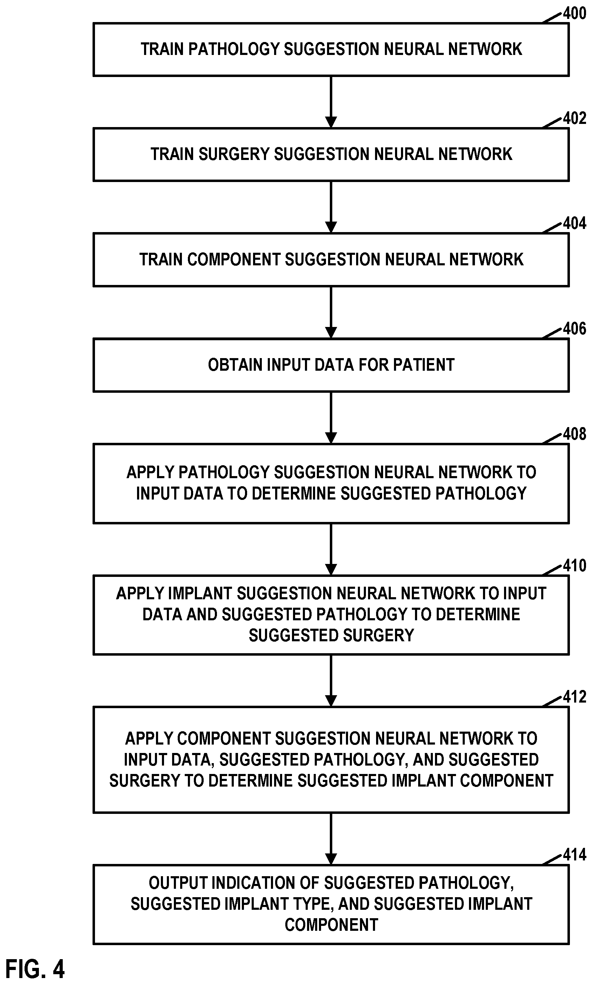

FIG. 4 is a flowchart illustrating an example operation for determining a suggested pathology, suggested surgery, and suggested implant component type, in accordance with one or more aspects of this disclosure. In the example of FIG. 4, computing system 102 may train pathology suggestion neural network 200 (400). Additionally, the computing system may train surgery suggestion neural network 202 (402). The computing system may also train component suggestion neural networks 204 (404). A discussion of training pathology suggestion neural network 200, surgery suggestion neural network 202, and component suggestion neural networks 204 is provided elsewhere in this disclosure.

Furthermore, in the example of FIG. 4, computing system 102 may obtain input data 206 for a patient (406). For instance, computing system 102 may retrieve all or part of input data 206 from an electronic medical record or other electronic storage system. In some examples, computing system 102 may automatically generate one or more parts of input data 206 based on analysis of medical imaging data. In some examples, computing system 102 may receive indications of user input of one or more parts of input data 206. In some examples, computing system 102 may obtain one or more parts of input data 206 from client devices 106.

Computing system 102 may then apply pathology suggestion neural network 200 to input data 206 to determine a suggested pathology for the patient (408). Computing system 102 may apply surgery suggestion neural network 202 to input data 206 and data indicating the suggested pathology to determine a suggested surgery for the patient (410). Computing system 102 may apply one of component suggestion neural networks 204 to input data 206, data indicating the suggested pathology, and data indicating the suggested surgery to determine a suggested implant component type for the patient (412).

Computing system 102 may output an indication of the suggested pathology, the suggested surgery, and/or a suggested implant component (414). Computing system 102 may output the indication of the suggested pathology, the suggested surgery, and suggested implant component in one or more of various ways. For example, computing system 102 may output the indication of at least one of the suggested pathology, the suggested surgery, or the suggested implant component for display on a display screen of computing system 102. In some examples, computing system 102 may output the indication of at least one of the suggested pathology, the suggested surgery, or the suggested implant component to one or more of client devices 106 (FIG. 1). In such examples, client devices 106 may output an indication of the suggested pathology, the suggested surgery, or the suggested implant component for display. In other examples, computing system 102 or client devices 106 may output audible indications of the suggested pathology, the suggested surgery, and/or the suggested implant component.

The indication of the suggested pathology may include text describing or naming the suggested pathology, a numerical index corresponding to the suggested pathology, or other data that identifies the suggested pathology. The indication of the suggested surgery may include text describing or naming the suggested surgery, a numerical index corresponding to the suggested surgery, or other data that identifies the suggested surgery. The indication of the suggested implant component may include text describing or naming the suggested implant, a part number of the suggested implant, or other data that identifies the suggested implant component.

As noted above, computing system 102 may train pathology suggestion neural network 200, surgery suggestion neural network 202, and component suggestion neural networks 204. When training a neural network such as pathology suggestion neural network 200, surgery suggestion neural network 202, or component suggestion neural networks 204, computing system 102 may perform multiple iterations of a training process for the neural network. During each iteration of the training process for the neural network, computing system 102 may provide a training dataset as input to the neural network. Computing system 102 may apply the neural network to the training dataset to generate an output vector corresponding to the training dataset. Computing system 102 may then apply a cost function (i.e., a loss function) to determine a cost value based on the output vector of the neural network and a target vector for the training dataset. Computing system 102 may apply one of various cost functions to determine the cost value. For example, computing system 102 may apply a mean-squared error function or another type of cost function to determine the cost value. After determining the cost value, computing system 102 may apply a backpropagation algorithm that may update the weights of inputs to individual neurons in the neural network.

As noted elsewhere in this disclosure, each output layer neuron in the plurality of output layer neurons corresponds to a different output element in a plurality of output elements. In examples where the neural network is pathology suggestion neural network 200, each output layer neuron may correspond to a different type of shoulder pathology. In examples where the neural network is surgery suggestion neural network 202, each output layer neuron may correspond to a different type of shoulder surgery. In examples where the neural network is one of component suggestion neural networks 204, each output layer neuron may correspond to a different type of implant component. Each output element in the plurality of output elements of the neural network corresponds to a different element pathology in an output vector. In this example, computing system 102 may generate a plurality of training datasets from past shoulder surgery cases. Each respective training dataset corresponds to a different training data patient in a plurality of training data patients and comprises a respective training input vector and a respective target output vector.

For each respective training dataset, the training input vector of the respective training dataset comprises a value for each element of the plurality of input elements (e.g., each element of input data 206). For each respective training dataset, the target output vector of the respective training dataset comprises a value for each element of the plurality of output elements. In this example, computing system 102 may use the plurality of training datasets to train the neural network. Additionally, in this example, computing system 102 may obtain a current input vector that corresponds to a current patient. The computing system may apply pathology suggestion neural network 200 to the current input vector to generate a current output vector. Computing system 102 may then determine, based on the current output vector, a classification of a shoulder condition of the current patient, which also may be referred to as a shoulder classification.

In accordance with a technique of this disclosure, computing system 102 trains pathology suggestion neural network 200, surgery suggestion neural network 202, and each of component suggestion neural networks 204 separately. For example, computing system 102 may train pathology suggestion neural network 200 through a process that reduces differences between output of pathology suggestion neural network 200 and target output values specifying target values for pathologies. In this example, computing system 102 may train surgery suggestion neural network 202 through a process that reduces differences between output of surgery suggestion neural network 202 and target output values for surgeries. Furthermore, in this example, computing system 102 may train each of component suggestion neural networks 204 through a process that reduces differences between output of the component suggestion neural network and target output values for an implant component type.

As noted above, computing system 102 may use a plurality of training datasets to train a neural network, such as pathology suggestion neural network 200, surgery suggestion neural network 202, and component suggestion neural networks 204. Each respective training dataset may correspond to a different training data patient in a plurality of training data patients. For instance, a first training dataset may correspond to a first training data patient, a second training dataset may correspond to a second training data patient, and so on. A training dataset may correspond to a training data patient in the sense that the training dataset may include information regarding the patient. The training data patients may be real patients. In some examples, the training data patients may include simulated patients.

Each respective training dataset may include a respective training input vector and a respective target output vector. For each respective training dataset, the training input vector of the respective training dataset comprises a value for each element of the plurality of input elements. In other words, the training input vector may include a value for each input layer neuron of the neural network. For each respective training dataset, the target output vector of the respective training dataset may comprise a value for each element of the plurality of output elements. In other words, the target output vector may include a value for each output layer neuron of the neural network.

In some examples, the values in a target output vector are binary (e.g., 0 or 1). For instance, in a target output vector used with pathology suggestion neural network 200, the target output vector may indicate 0 for all pathologies except for a pathology that a surgeon identified for the corresponding training data patient, which may have a value of 1. Likewise, in a target output vector used with surgery suggestion neural network 202, the target output vector may indicate 0 for all surgeries except for a surgery that a surgeon identified for treating the corresponding training data patient, which may have a value of 1. In a target output vector used with a component suggestion neural network, the target output vector may indicate 0 for all implant components except for an implant component that a surgeon identified for the corresponding training data patient, which may have a value of 1.

In some examples, the values in a target output vector are confidence values. Such confidence values may, in turn, be based on levels of confidence expressed by one or more trained healthcare professionals, such as orthopedic surgeons. For instance, a trained healthcare professional may be given the information in the training input vector of a training dataset (or information from which the training input vector of the training dataset is derived) and may be asked to provide a confidence level that the training data patient has a pathology belonging to each pathology in a set of pathologies. For instance, in an example where the set of pathologies includes PGHOA, RCTA instability, MRCT, and rheumatoid arthritis, the healthcare professional may indicate that their level of confidence that the training data patient's pathology is PGHOA is 0 (meaning she does not at all believe that the training data patient's pathology is PGHOA), indicate that their level of confidence that the training data patient's pathology is RCTA instability is 0; indicate that their level of confidence that the training data patient's pathology is MRCT is 0.75 (meaning they are fairly confident that the training data patient's pathology is MRCT); indicate that their level of confidence that the training data patient's pathology is rheumatoid arthritis is 0.25 (meaning she believes that there is a smaller chance that the training data patient's pathology is rheumatoid arthritis). Similar examples may be provided with respect to surgeries and implant components. In some examples, computing system 102 may apply the inverse of the confidence value function to the confidence values provided by the healthcare professional to generate values to include in the target output vector. In some examples, the confidence values provided by the healthcare professional are the values included in the target output vector.

Different healthcare professionals may have different levels of confidence that the same training data patient has a pathology belonging to each pathology in a plurality of pathologies. Likewise, different healthcare professionals may have different levels of confidence that the same training data patient should have the same surgery. Similarly, different healthcare professionals may have different levels of confidence that the same training data patient should have a particular implant component in a set of implant components. Hence, in some examples, the confidence values upon which the values in a target output vector are based may be averages or otherwise determined from the confidence levels provided by multiple healthcare professionals.

In some such examples, the confidence levels of some healthcare professionals may be given greater weight in a weighted average of confidence levels than the confidence levels of other healthcare professionals. For instance, the confidence levels of a preeminent orthopedic surgeon may be given greater weight than the confidence levels of other orthopedic surgeons. In another example, the confidence levels of healthcare professionals or training data patients in particular regions or hospitals may be given greater weight than healthcare professionals or training data patients from other regions or hospitals. Advantageously, such weighted averaging may allow the neural network (e.g., pathology suggestion neural network 200, surgery suggestion neural network 202, component suggestion neural networks 204) to be tuned according to various criteria and preferences.

For instance, a healthcare professional may prefer to use a neural network that has been trained such that confidence levels are weighted in particular ways. In some examples where training datasets include training datasets based on a healthcare professional's own cases, the healthcare professional (e.g., an orthopedic surgeon) may prefer to use a neural network trained using training datasets where the healthcare professional's own cases are weighted more heavily or exclusively using the healthcare professional's own cases. In this way, the neural network may generate output tailored to the healthcare professional's own style of practice. For instance, the neural network may be more likely to output indicates of surgeries or implant components preferred by the healthcare professional. Moreover, healthcare professionals and patients may have difficulty trusting the output of a computing system. Accordingly, in some examples, computing system 102 may provide information indicating that the neural network was trained to emulate the decisions of the healthcare professionals themselves and/or particularly trusted orthopedic surgeons.

In some examples, the confidence levels of different healthcare professionals for the same training data patient may be used in generating different training datasets. For instance, the confidence levels of a first healthcare professional with respect to a particular training data patient may be used to generate a first training dataset and the confidence levels of a second healthcare professional with respect to the same training data patient may be used to generate a second training dataset.

Furthermore, in some examples, computing system 102 may provide confidence values for output to one or more users. For instance, computing system 102 may provide the confidence values to client devices 106 for display to one or more users. In this way, the one or more users may be better able to understand how computing system 102 may have arrived at the suggested pathology, suggested surgery, and suggested implant components.

In some examples, as part of generating the training datasets, computing system 102 may select the plurality of training datasets from a database of training datasets based on one or more training dataset selection criteria. In other words, computing system 102 may exclude certain training datasets from the training process of the neural network if the training datasets do not satisfy the training dataset selection criteria.

There may be a wide variety of training dataset selection criteria. For instance, in one example, the one or more training data set selection criteria may include which surgeon operated on the plurality of training data patients. In some examples, the one or more training dataset selection criteria include a geographic region in which the training data patients live. In some examples, the one or more training dataset selection criteria include a geographic region associated with one or more surgeons (e.g., a region in which the one or more surgeons practice, live, were licensed, were trained, etc.).

In some examples, the one or more training dataset selection criteria include postoperative health outcomes of the training data patients. In such examples, the postoperative health outcomes of the training data patients may include one or more of: postoperative range of motion, presence of postoperative infection, or postoperative pain. Thus, in such examples, the training datasets upon which the neural network is trained may exclude training datasets where adverse health outcomes occurred.

Additional training datasets may be added to the database over time and computing system 102 may use the additional training datasets to train pathology suggestion neural network 200, surgery suggestion neural network 202, and component suggestion neural networks 204. Thus, pathology suggestion neural network 200, surgery suggestion neural network 202, and component suggestion neural networks 204 may continue to improve over time as more training datasets are added to training data 120.

Although the techniques of this disclosure have been described with respect to a cascade of artificial neural networks, specific examples of this disclosure may be implemented in which one or more of the artificial neural networks are replaced by other types of machine learning models, such as support vector machines (SVMs) or random forest models. For instance, in the example of FIG. 2, one or more of pathology suggestion neural network 200, surgery suggestion neural network 202, or one or more of component suggestion neural network(s) 204 may be replaced with another type of machine learning model that performs a similar function.

When a machine learning model is implemented using an SVM, training data 120 (FIG. 1) includes a set of samples. Each sample of training data 120 includes an input vector and a target classification. The input vector includes a set of features. The features in the input vector may include data for a specific patient, and in some examples, may also include other types of data regarding the specific patient. The target classification is a pre-determined classification associated with the specific patient.

Furthermore, computing system 102 may determine a set of weights w based on training data 120. The set of weights w includes a weight for each feature of the input vector. Furthermore, computing system 102 may determine whether an input vector belongs to a first classification or a second classification, and hence, whether a patient associated with the input vector is associated with the first classification or the second classification, by applying the following formula:

$$\hat{y} = \begin{array}{l} 0 \text{ if } \mathbf{w}^T \cdot \mathbf{x} + b < 0 \\ 1 \text{ if } \mathbf{w}^T \cdot \mathbf{x} + b \geq 0 \end{array}$$

In the equation above, x denotes the input vector, b denotes a bias term, and $\hat{y}$ is an index of the class.

Furthermore, in examples where computing system 102 implements an SVM algorithm, computing system 102 may determine the weights w and the bias term b using linear SVM classification or nonlinear SVM classification. Training an SVM classifier entails determining values of $\mathbf{w}$ and $b$ that maximize a margin between two lines (i.e., support vectors) that are parallel to a linear decision boundary while avoiding or limiting margin violations (i.e., input vectors that are between the support vectors). In some examples, computing system 102 may use a stochastic gradient descent algorithm, a sequential minimal optimization algorithm, or another algorithm to determine the values of $\mathbf{w}$ and $b$.

In some examples, to use the sequential minimal optimization algorithm, computing system 102 may solve the quadratic programming problem expressed by:

$$\max_{\alpha} \sum_{i=1}^{n} \alpha_i - \frac{1}{2} \sum_{i=1}^{n} \sum_{j=1}^{n} y_i y_j K(x_i, x_j) \alpha_i \alpha_j,$$

subject to:

$$0 \leq \alpha_i \leq C, \text{ for } i = 1, 2, \dots, n$$

and

$$\sum_{i=1}^{n} y_i \alpha_i = 0$$

In the equations above, $n$ indicates a number of samples in the dataset, $y_i$ and $y_j$ indicate target classifications of the samples, $x_i$ and $x_j$ indicate input vectors of the samples, $\alpha_i$ and $\alpha_j$ are Lagrange multipliers, $K(x_i, x_j)$ is a kernel function, and $C$ is a SVM hyperparameter. Computing system 102 may use various kernel functions as $K(x_i, x_j)$, such as a linear kernel function, a polynomial kernel function, a Gaussian Radial Basis Function (RBF) kernel function, a sigmoid kernel function, or another type of kernel function.

To solve the quadratic programming problem expressed above, computing system 102 may find a Lagrange multiplier $\alpha_1$ that violates the Karush-Kuhn-Tucker (KKT) conditions for the optimization problem. Computing system 102 may then determine a second Lagrange multiplier $\alpha_2$ and optimize the pair $(\alpha_1, \alpha_2)$. Computing system 102 may repeat these steps until convergence. When the Lagrange multipliers satisfy the KKT conditions within a tolerance (e.g., a user-defined tolerance), the sequential minimal optimization problem is solved. Computing system 102 may determine the set of weights **w** and the bias term *b* as:

$$\mathbf{w} = \sum_{i=1}^{n} \alpha_i y_i x_i$$

and

$$b = \mathbf{w} \cdot x_i - y_i$$

In another example, computing system 102 may implement a machine learning model using a random forest algorithm. In this example, computing system 102 may generate a plurality of decision trees using randomly selected samples from training data 122. In other words, for each decision tree in the plurality of decision trees, computing system 102 may generate a tree-specific training dataset that is a subset of the samples in training data 122. In some examples, computing system 102 may use bagging or pasting to select the samples in the tree-specific training datasets for the plurality of decision trees. In examples where the classifier algorithm is implemented using a random forest algorithm, each sample of training data 122 includes an input vector and a target classification. The input vector includes a set of features. The features in the input vector may include bony landmark data for a specific patient, and in some examples, may also include other types of data regarding the specific patient. The target classification is a pre-determined classification associated with the specific patient.

Furthermore, in examples where the classifier algorithm is implemented using a random forest algorithm, each of the decision trees outputs a proposed classification associated with the patient. Computing system 102 may then select one of the proposed classifications as the classification associated with the patient. For example, computing system 102 may use a voting system in which computing system 102 selects, as the classification associated with the patient, a most common one of the proposed classifications.

In examples where the classifier algorithm is implemented using a random forest algorithm, computing system 102 may generate the decision trees using one or more of a variety of decision tree training algorithms. For instance. in some examples, computing system 102 generates the decision tress using a Classification and Regression Tree (CART) algorithm. To perform the CART algorithm, computing system 102 may split the tree-specific training data subset into two subsets using a single feature *k* and a threshold $t_k$. To select feature *k* and threshold $t_k$, computing system 102 may search for a pair $(k, t_k)$ that produces the "purest" subsets of the tree-specific training data set. A subset is considered "pure" if all of the target classifications in the subset are the same. Example measures for determining the purity of a subset include a Gini impurity measure, an entropy impurity measure, and so on. The following equation is an example cost function that surgical assistance system 100 may use to generate cost values that surgical assistance system 100 may use for comparing combinations of pairs $(k, t_k)$.

$$J(k, t_k) = \frac{m_{left}}{m} G_{left} + \frac{m_{right}}{m} G_{right}$$

In the equation above, $G_{left/right}$ is a measure of the impurity of the left/right subset, and $m_{left/right}$ is a number of instances in the left/right subset. The "left" and "right" subsets are so named because they may correspond to left and right branches of a decision tree.

In the CART algorithm, after selecting a pair $(k, t_k)$ that splits the tree-specific training data subset into two subsets, computing system 102 may further divide these two subsets in the same manner as described above. Computing system 102 may continue recursively dividing subsets in this manner using a maximum depth is reached or surgical assistance system 100 is unable to determine any combination of features and thresholds that reduce impurity within a subset.

While the techniques been disclosed with respect to a limited number of examples, those skilled in the art, having the benefit of this disclosure, will appreciate numerous modifications and variations there from. For instance, it is contemplated that any reasonable combination of the described examples may be performed. It is intended that the appended claims cover such modifications and variations as fall within the true spirit and scope of the invention.

It is to be recognized that depending on the example, certain acts or events of any of the techniques described herein can be performed in a different sequence, may be added, merged, or left out altogether (e.g., not all described acts or events are necessary for the practice of the techniques). Moreover, in certain examples, acts or events may be performed concurrently, e.g., through multi-threaded processing, interrupt processing, or multiple processors, rather than sequentially.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transitory media, but are instead directed to non-transitory, tangible storage media. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Operations described in this disclosure may be performed by one or more processors, which may be implemented as fixed-function processing circuits, programmable circuits, or combinations thereof, such as one or more digital signal processors (DSPs), general purpose microprocessors, application specific integrated circuits (ASICs), field programmable gate arrays (FPGAs), or other equivalent integrated or discrete logic circuitry. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute instructions specified by software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. Accordingly, the terms "processor" and "processing circuity," as used herein may refer to any of the foregoing structures or any other structure suitable for implementation of the techniques described herein.

Various examples have been described. These and other examples are within the scope as defined by the following claims.

## Claims

1. A computer-implemented method for determining a suggested implant component to be implanted into a patient during an orthopedic surgery, the method comprising:

    applying, by a computing system, a first machine learning model to determine a suggested pathology, wherein an input vector of the first machine learning model includes a set of input data, the set of input data including anatomic parameters of the patient;
    applying, by the computing system, a second machine learning model to determine a suggested surgery, wherein an input vector of the second machine learning model includes an element that indicates the suggested pathology;
    applying, by the computing system, a third machine learning model to determine the suggested implant component to implant into the patient during the orthopedic surgery, wherein an input vector of the third machine learning model includes an element that indicates the suggested pathology and an element that indicates the suggested surgery; and
outputting, by the computing system, an indication of the suggested implant component.

2. The method of claim 1, wherein:

   the input vector of the second machine learning model includes the input data, and
   the input vector of the third machine learning model includes the input data.

3. The method of any of claims 1-2, further comprising:

   training, by the computing system, the first machine learning model;
   training, by the computing system, the second machine learning model; and
   training, by the computing system, the third machine learning model,
   wherein the computing system trains the first machine learning model, the second machine learning model, and
   the third machine learning model separately from each other.

4. The method of any of claims 1-3, wherein the suggested implant component is a first suggested implant component, the first suggested implant component belongs to a first implant component type, the method further comprising: applying, by the computing system, a fourth machine learning model to determine a second suggested implant component to be implanted into the patient during the orthopedic surgery, wherein the fourth machine learning model is separate from the third machine learning model, the second suggested implant component belongs to a second implant component type, and the first and second suggested implant components are designed for attachment to different bones of the patient.

5. The method of claim 4, wherein the orthopedic surgery is a shoulder repair surgery, the first suggested implant component is a glenoid implant and the second suggested implant component is a humeral implant component.

6. The method of any of claims 1-5, wherein:

   the first machine learning model is a first artificial neural network,
   the first machine learning model comprises an output layer, wherein different neurons in the output layer of the first machine learning model correspond to different pathologies in a plurality of pathologies, and
   the plurality of pathologies includes two or more of: primary glenoid humeral osteoarthritis, rotator cuff tear arthropathy instability, massive rotator cuff tear, rheumatoid arthritis, post-traumatic arthritis, or osteoarthritis.

7. The method of any of claims 1-6, wherein:

   the second machine learning model is a second artificial neural network,
   the second machine learning model comprises an output layer, wherein different neurons in the output layer of the second machine learning model correspond to different orthopedic surgeries in a plurality of orthopedic surgeries, and
   the plurality of orthopedic surgeries includes two or more of: anatomical total shoulder arthroplasty, reverse total shoulder arthroplasty, or shoulder hemiarthroplasty.

8. The method of any of claims 1-7, wherein the anatomic parameters include parameters corresponding to at least one of: glenoid wear orientation, glenoid bone loss, humeral bone loss, a Hill-Sachs lesion, or a Bankart lesion.

9. The method of any of claims 1-8, wherein the input data includes one or more bony landmark metrics, the bony landmark metrics being numerical values characterizing distances or angles between landmarks on one or more bones of the patient.

10. The method of claim 9, wherein the bony landmark metrics include one or more of: a distance between a humerus of the patient and a glenoid of the patient, a distance between an acromion of the patient and a humeral head of the patient, a glenoid coracoid process angle, or an infra-glenoid tubercle angle.

11. The method of any of claims 1-10, wherein:

    the third machine learning model is a third artificial neural network,

the third machine learning model comprises an output layer, wherein different neurons in the output layer of the third machine learning model correspond to different implant components.

12. The method of any of claims 1-11, wherein the first, second, and third machine learning models are separate artificial neural networks.

13. The method of any of claims 1-11, wherein one or more of the first, second, and third machine learning models is a support vector machine or a random forest model.

14. A computing system comprising means for performing the methods of any of claims 1-13.

**Patentansprüche**

1. Computerimplementiertes Verfahren zum Bestimmen einer empfohlenen Implantatkomponente, die in einen Patienten während einer orthopädischen Operation implantiert werden soll, das Verfahren umfassend:

Anwenden, durch ein Computersystem, eines ersten Maschinenlernmodells zum Bestimmen einer empfohlenen Pathologie, wobei ein Eingabevektor des ersten Maschinenlernmodells einen Satz von Eingabedaten umfasst, wobei der Satz von Eingabedaten anatomische Parameter des Patienten umfasst;
Anwenden, durch das Computersystem, eines zweiten Maschinenlernmodells zum Bestimmen einer empfohlenen Operation, wobei ein Eingabevektor des zweiten Maschinenlernmodells ein Element umfasst, das die empfohlene Pathologie angibt;
Anwenden, durch das Computersystem, eines dritten Maschinenlernmodells zum Bestimmen der empfohlenen Implantatkomponente zum Implantieren in den Patienten während der orthopädischen Operation, wobei ein Eingabevektor des dritten Maschinenlernmodells umfasst: ein Element, das die empfohlene Pathologie angibt, und ein Element, das die empfohlene Operation angibt; und
Ausgeben, durch das Computersystem, einer Angabe der empfohlenen Implantatkomponente.

2. Verfahren nach Anspruch 1, wobei:

der Eingabevektor des zweiten Maschinenlernmodells die Eingabedaten umfasst, und
der Eingabevektor des dritten Maschinenlernmodells die Eingabedaten umfasst.

3. Verfahren nach einem der Ansprüche 1 bis 2, ferner umfassend:

Trainieren, durch das Computersystem, des ersten Maschinenlernmodells;
Trainieren, durch das Computersystem, des zweiten Maschinenlernmodells; und
Trainieren, durch das Computersystem, des dritten Maschinenlernmodells,
wobei das Computersystem das erste Maschinenlernmodell, das zweite Maschinenlernmodell und das dritte Maschinenlernmodell getrennt voneinander trainiert.

4. Verfahren nach einem der Ansprüche 1 bis 3, wobei die empfohlene Implantatkomponente eine erste empfohlene Implantatkomponente ist, die erste empfohlene Implantatkomponente zu einer ersten Implantatkomponentenart gehört, das Verfahren ferner umfassend:
Anwenden, durch das Computersystem, eines vierten Maschinenlernmodells zum Bestimmen einer zweiten empfohlenen Implantatkomponente, die in den Patienten während der orthopädischen Operation implantiert werden soll, wobei das vierte Maschinenlernmodell von dem dritten Maschinenlernmodell getrennt ist, die zweite empfohlene Implantatkomponente zu einer zweiten Implantatkomponentenart gehört und die erste und die zweite empfohlene Implantatkomponente für eine Befestigung an unterschiedlichen Knochen des Patienten ausgelegt sind.

5. Verfahren nach Anspruch 4, wobei die orthopädische Operation eine Schulterreparaturoperation ist, die erste empfohlene Implantatkomponente ein Glenoidimplantat ist und die zweite empfohlene Implantatkomponente eine Humerusimplantatkomponente ist.

6. Verfahren nach einem der Ansprüche 1 bis 5, wobei:

das erste Maschinenlernmodell ein erstes künstliches neuronales Netzwerk ist,

das erste Maschinenlernmodell eine Ausgabeschicht umfasst, wobei unterschiedliche Neuronen in der Ausgabeschicht des ersten Maschinenlernmodells mit unterschiedlichen Pathologien in einer Vielzahl von Pathologien korrespondieren, und

die Vielzahl von Pathologien zwei oder mehr umfasst von: primärer Glenoidhumerusosteoarthritis, Rotatorenmanschettenriss-Arthropathie-Instabilität, massivem Rotatorenmanschettenriss, rheumatoider Arthritis, posttraumatischer Arthritis oder Osteoarthritis.

7. Verfahren nach einem der Ansprüche 1 bis 6, wobei:

das zweite Maschinenlernmodell ein zweites künstliches neuronales Netzwerk ist,

das zweite Maschinenlernmodell eine Ausgabeschicht umfasst, wobei unterschiedliche Neuronen in der Ausgabeschicht des zweiten Maschinenlernmodells mit unterschiedlichen orthopädischen Operationen in einer Vielzahl von orthopädischen Operationen korrespondieren, und

die Vielzahl von orthopädischen Operationen zwei oder mehr umfasst von: anatomischer Totalschulterarthroplastik, inverser Totalschulterarthroplastik oder Schulterhem iarthroplastik.

8. Verfahren nach einem der Ansprüche 1 bis 7, wobei die anatomischen Parameter Parameter umfassen, die mindestens einem entsprechen von:
Glenoidabnutzungsausrichtung, Glenoidknochenverlust, Humerusknochenverlust, Hill-Sachs-Läsion oder Bankart-Läsion.

9. Verfahren nach einem der Ansprüche 1 bis 8, wobei die Eingabedaten eine oder mehrere knöcherne Landmarkenmetriken umfassen, wobei die knöchernen Landmarkenmetriken numerische Werte sind, die Abstände oder Winkel zwischen Landmarken auf einem oder mehreren Knochen des Patienten kennzeichnen.

10. Verfahren nach Anspruch 9, wobei die knöchernen Landmarkenmetriken einen oder mehrere umfassen von: einem Abstand zwischen einem Humerus des Patienten und einem Glenoid des Patienten, einem Abstand zwischen einem Akromion des Patienten und einem Humeruskopf des Patienten, einem Glenoidrabenschnabelfortsatzwinkel oder einem Infra-Glenoidtuberkelwinkel.

11. Verfahren nach einem der Ansprüche 1 bis 10, wobei:

das dritte Maschinenlernmodell ein drittes künstliches neuronales Netzwerk ist,

das dritte Maschinenlernmodell eine Ausgabeschicht umfasst, wobei unterschiedliche Neuronen in der Ausgabeschicht des dritten Maschinenlernmodells mit unterschiedlichen Implantatkomponenten korrespondieren.

12. Verfahren nach einem der Ansprüche 1 bis 11, wobei das erste, das zweite und das dritte Maschinenlernmodell separate künstliche neuronale Netzwerke sind.

13. Verfahren nach einem der Ansprüche 1 bis 11, wobei eines oder mehrere des ersten, des zweiten und des dritten Maschinenlernmodells eine Support-Vector-Maschine oder ein Random-Forest-Modell sind.

14. Computersystem, umfassend Mittel zum Durchführen der Verfahren nach einem der Ansprüche 1 bis 13.

**Revendications**

1. Procédé mis en œuvre par ordinateur pour déterminer un composant d'implant proposé pour une implantation dans un patient pendant une chirurgie orthopédique, le procédé comprenant :

l'application, par un système informatique, d'un premier modèle d'apprentissage machine pour déterminer une pathologie proposée, dans lequel un vecteur d'entrée du premier modèle d'apprentissage machine inclut un ensemble de données d'entrée, l'ensemble de données d'entrée incluant des paramètres anatomiques du patient ;

l'application, par le système informatique, d'un deuxième modèle d'apprentissage machine pour déterminer une pathologie proposée, dans lequel un vecteur d'entrée du deuxième modèle d'apprentissage machine inclut un élément qui indique la pathologie proposée ;

l'application, par le système informatique, d'un troisième modèle d'apprentissage machine pour déterminer le composant d'implant proposé pour une implantation dans le patient pendant la chirurgie orthopédique, dans lequel un vecteur d'entrée du troisième modèle d'apprentissage machine inclut un élément qui indique la pathologie proposée et un élément qui indique la chirurgie proposée ; et

la sortie, par le système informatique, d'une indication du composant d'implant proposé.

2. Procédé selon la revendication 1, dans lequel :

le vecteur d'entrée du deuxième modèle d'apprentissage machine inclut la donnée d'entrée, et
le vecteur d'entrée du troisième modèle d'apprentissage machine inclut la donnée d'entrée.

3. Procédé selon l'une quelconque des revendications 1-2, comprenant en outre :

l'entraînement, par le système informatique, du premier modèle d'apprentissage machine ;
l'entraînement, par le système informatique, du deuxième modèle d'apprentissage machine ; et
l'entraînement, par le système informatique, du troisième modèle d'apprentissage machine,
dans lequel le système informatique entraîne le premier modèle d'apprentissage machine, le deuxième modèle d'apprentissage machine et le troisième modèle d'apprentissage machine séparément les uns des autres.

4. Procédé selon l'une quelconque des revendications 1-3, dans lequel le composant d'implant proposé est un premier composant d'implant proposé, le premier composant d'implant proposé appartient à un premier type de composant d'implant, le procédé comprenant en outre :
l'application, par le système informatique, d'un quatrième modèle d'apprentissage machine pour déterminer un deuxième composant d'implant proposé pour une implantation dans le patient pendant la chirurgie orthopédique, dans lequel le quatrième modèle d'apprentissage machine est séparé du troisième modèle d'apprentissage machine, le deuxième composant d'implant proposé appartient à un deuxième type de composant d'implant, et les premier et deuxième composants d'implant proposés sont conçus pour être fixés à des os différents du patient.

5. Procédé selon la revendication 4, dans lequel la chirurgie orthopédique est une chirurgie de réparation d'épaule, le premier composant d'implant proposé est un implant glénoïdien et le deuxième composant d'implant proposé est un composant d'implant huméral.

6. Procédé selon l'une quelconque des revendications 1-5, dans lequel :

le premier modèle d'apprentissage machine est un premier réseau neuronal artificiel,
le premier modèle d'apprentissage machine comprend une couche de sortie, dans lequel des neurones différents dans la couche de sortie du premier modèle d'apprentissage machine correspondent à des pathologies différentes parmi une pluralité de pathologies, et
la pluralité de pathologies inclut deux ou plusieurs parmi : une ostéoarthrite gléno-humérale primaire, une instabilité d'arthrite psoriasique avec rupture de la coiffe des rotateurs, une rupture massive de la coiffe des rotateurs, une polyarthrite rhumatoïde, une arthrite post-traumatique ou une ostéoarthrite.

7. Procédé selon l'une quelconque des revendications 1-6, dans lequel :

le deuxième modèle d'apprentissage machine est un deuxième réseau neuronal artificiel,
le deuxième modèle d'apprentissage machine comprend une couche de sortie, dans lequel des neurones différents dans la couche de sortie du deuxième modèle d'apprentissage machine correspondent à des chirurgies orthopédiques différentes parmi une pluralité de chirurgies orthopédiques, et
la pluralité de chirurgies orthopédiques inclut deux ou plusieurs parmi : une arthroplastie totale d'épaule anatomique, une arthroplastie totale d'épaule inversée ou une hémiarthroplastie d'épaule.

8. Procédé selon l'une quelconque des revendications 1-7, dans lequel les paramètres anatomiques incluent des paramètres correspondant à au moins l'un parmi : une orientation d'usure glénoïdienne, une perte d'os glénoïdien, une perte d'os huméral, une lésion de Hill-Sachs ou une lésion de Bankart.

9. Procédé selon l'une quelconque des revendications 1-8, dans lequel la donnée d'entrée inclut une ou plusieurs mesures de repère osseux, les mesures de repère osseux étant des valeurs numériques caractérisant des distances ou des angles entre des repères sur un ou plusieurs os du patient.

**10.** Procédé selon la revendication 9, dans lequel les mesures de repère osseux incluent un ou plusieurs parmi : une distance entre un humérus du patient et une glénoïde du patient, une distance entre un acromion du patient et une tête humérale du patient, un angle de processus coracoïde glénoïdien ou un angle de tubercule infraglénoïdal.

**11.** Procédé selon l'une quelconque des revendications 1-10, dans lequel :

le troisième modèle d'apprentissage machine est un troisième réseau neuronal artificiel,
le troisième modèle d'apprentissage machine comprend une couche de sortie, dans lequel des neurones différents dans la couche de sortie du troisième modèle d'apprentissage machine correspondent à des composants d'implant différents.

**12.** Procédé selon l'une quelconque des revendications 1-11, dans lequel les premier, deuxième et troisième modèles d'apprentissage machine sont des réseaux neuronaux artificiels séparés.

**13.** Procédé selon l'une quelconque des revendications 1-11, dans lequel un ou plusieurs parmi les premier, deuxième et troisième modèles d'apprentissage machine sont une machine à vecteurs de support ou un modèle de forêt d'arbres décisionnels.

**14.** Système informatique comprenant des moyens pour exécuter les procédés selon l'une quelconque des revendications 1-13.

100

COMPUTING SYSTEM
102

PROCESSING
CIRCUIT(S)
108

DATA STORAGE SYSTEM
110

TRAINING DATA
120

PATHOLOGY
SUGGESTION NN
PARAMETERS
114

SURGERY
SUGGESTION NN
PARAMETERS
116

COMPONENT
SUGGESTION NN
PARAMETERS
118

COMMUNICATION
INTERFACE(S)
112

104

COMMUNICATION
NETWORK

CLIENT DEVICE
106A

● ● ●

CLIENT DEVICE
106N

FIG. 1

INPUT DATA
206

PATHOLOGY SUGGESTION NEURAL NETWORK
200

*Suggested pathology*

SURGERY SUGGESTION NEURAL NETWORK
202

*Suggested surgery type*

*Suggested pathology*

COMPONENT SUGGESTION NEURAL NETWORK(S)
204

*Suggested pathology*

*Suggested surgical items*

*Suggested surgery type*

FIG. 2

300

Output vector ⎯310

304

306

302

Input vector ⎯308

FIG. 3

```
                                                                    ┌─400
        ┌──────────────────────────────────────────────────┐
        │      TRAIN PATHOLOGY SUGGESTION NEURAL NETWORK      │
        └──────────────────────────────────────────────────┘
                                │
                                ▼
                                                                    ┌─402
        ┌──────────────────────────────────────────────────┐
        │       TRAIN SURGERY SUGGESTION NEURAL NETWORK       │
        └──────────────────────────────────────────────────┘
                                │
                                ▼
                                                                    ┌─404
        ┌──────────────────────────────────────────────────┐
        │      TRAIN COMPONENT SUGGESTION NEURAL NETWORK      │
        └──────────────────────────────────────────────────┘
                                │
                                ▼
                                                                    ┌─406
        ┌──────────────────────────────────────────────────┐
        │            OBTAIN INPUT DATA FOR PATIENT            │
        └──────────────────────────────────────────────────┘
                                │
                                ▼
                                                                    ┌─408
        ┌──────────────────────────────────────────────────┐
        │    APPLY PATHOLOGY SUGGESTION NEURAL NETWORK TO     │
        │  INPUT DATA TO DETERMINE SUGGESTED PATHOLOGY        │
        └──────────────────────────────────────────────────┘
                                │
                                ▼
                                                                    ┌─410
        ┌──────────────────────────────────────────────────┐
        │  APPLY IMPLANT SUGGESTION NEURAL NETWORK TO INPUT  │
        │    DATA AND SUGGESTED PATHOLOGY TO DETERMINE        │
        │                SUGGESTED SURGERY                    │
        └──────────────────────────────────────────────────┘
                                │
                                ▼
                                                                    ┌─412
        ┌──────────────────────────────────────────────────┐
        │   APPLY COMPONENT SUGGESTION NEURAL NETWORK TO      │
        │  INPUT DATA, SUGGESTED PATHOLOGY, AND SUGGESTED     │
        │ SURGERY TO DETERMINE SUGGESTED IMPLANT COMPONENT   │
        └──────────────────────────────────────────────────┘
                                │
                                ▼
                                                                    ┌─414
        ┌──────────────────────────────────────────────────┐
        │    OUTPUT INDICATION OF SUGGESTED PATHOLOGY,       │
        │  SUGGESTED IMPLANT TYPE, AND SUGGESTED IMPLANT      │
        │                   COMPONENT                         │
        └──────────────────────────────────────────────────┘
```

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62910110
- US 2019146458 A1